(19) Europäisches Patentamt
European Patent Office
Office européen des brevets

(11) Veröffentlichungsnummer: 0 229 592
B1

# (12) EUROPÄISCHE PATENTSCHRIFT

(45) Veröffentlichungstag der Patentschrift: 18.10.89

(51) Int. Cl.⁴: C07C 45/44, C07C 47/548, C07F 5/06

(21) Anmeldenummer: 86810586.7

(22) Anmeldetag: 15.12.86

## (54) Verfahren zur Herstellung von 4,4'-Stilbendialdehyden.

(30) Priorität: 19.12.85 CH 5448/85

(43) Veröffentlichungstag der Anmeldung: 22.07.87 Patentblatt 87/30

(45) Bekanntmachung des Hinweises auf die Patenterteilung: 18.10.89 Patentblatt 89/42

(84) Benannte Vertragsstaaten: CH DE ES FR GB IT LI

(56) Entgegenhaltungen:
EP-A- 0 087 298
EP-A- 0 104 296
DE-A- 2 732 227

PATENT ABSTRACTS OF JAPAN, unexamined applications, C Sektion, Band 1, Nr. 12, 22. März 1977 THE PATENT OFFICE JAPANESE GOVERNMENT Seite 531 C76

(73) Patentinhaber: CIBA-GEIGY AG, Klybeckstrasse 141, CH-4002 Basel(CH)

(72) Erfinder: Reinehr, Dieter, Dr., Wolfsheule 10, D-7842 Kandern(DE)

EP 0 229 592 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues Verfahren zur Herstellung von 4,4'-Stilbendialdehyden der Formel

(1) 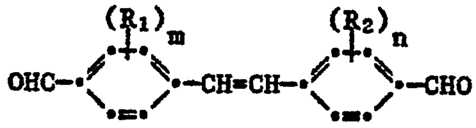 ,

worin $R_1$ und $R_2$ unabhängig voneinander Substituenten sind, die unter den für das erfindungsgemässe Verfahren charakteristischen Reatkionsbedingungen nicht reduziert werden, und m und n unabhängig voneinander 0, 1 oder 2 sind.

Das erfindungsgemässe Verfahren ist dadurch gekennzeichnet, dass man die Verbindungen der Formel

(2) 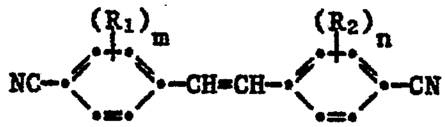 ,

worin $R_1$, $R_2$, m und n die angegebenen Bedeutungen haben, in einem wasserfreien, inerten, aprotischen organischen Lösungsmittel mit einem Aluminiumhydrid umsetzt, die erhaltene Reaktionsmischung anschliessend einer sauren Hydrolyse unterwirft und die Verbindungen der Formel (1) isoliert.

In den Verbindungen der Formel (1) bedeuten $R_1$ und $R_2$ unabhängig voneinander Substituenten, die unter den für das erfindungsgemässe Verfahren charakterischen Reaktionsbedingungen nicht reduziert werden können. Beispielsweise sind $R_1$ und $R_2$ Alkyl oder Alkoxy, insbesondere mit je 1 bis 4 Kohlenstoffatomen, Aryl oder Aryloxy, wie z.B. Phenyl und Phenoxy, Halogen, insbesondere Chlor und Brom, oder Dialkylamine mit vorzugsweise je 1 bis 4 Kohlenstoffatome in den Alkylresten.

Die Indices m und n sind unabhängig voneinander 0, 1 oder 2. Vorzugsweise bedeuten sie gleichzeitig 0.

Im erfindungsgemässen Verfahren werden die Verbindungen der Formel (2), worin $R_1$, $R_2$, m und n die angegebenen Bedeutungen haben, in einem wasserfreien, inerten, aprotischen organischen Lösungsmittel vorgelegt. Geeignete Lösungsmittel sind z.B. Kohlenwasserstoffe wie Alkane und Cycloalkane mit je 5 bis 10 Kohlenstoffatomen, beispielsweise Pentan, Hexan, Heptan, Octan, Nonan, Decan, Cyclopentan, Cyclohexan, Methylcyclohexan, Cyclooctan und Dekalin, sowie Aromaten wie Benzol und Toluol; und Aether wie Diäthyl- oder Dibutyläther, Dioxan oder Tetrahydrofuran, sowie Gemische dieser Lösungsmittel.

Zu dieser Lösung bzw. Dispersion der Verbindung der Formel (2) fügt man langsam ein Aluminiumhydrid, gelöst in vorzugsweise demselben Lösungsmittel wie die Verbindung der Formel (2), hinzu. Für das erfindungsgemässe Verfahren geeignete Aluminiumhydride entsprechen z.B. den Formeln $R_3R_4AlH$, $M[R_3R_4AlH_2]$ oder $MAlH_4$, worin $R_3$ und $R_4$ unabhängig voneinander Alkyl mit je 1 bis 4, vorzugweise 2 oder 4 Kohlenstoffatomen sind, und M Lithium oder Natrium ist. Bevorzugte Aluminiumhydride entsprechen den Formeln $(^iC_4H_9)_2AlH$, $Na[^i(C_4H_9)_2AlH_2]$ und $LiAlH_4$, wobei mit $(^iC_4H_9)_2$ AlH ganz besonders gute Ergebnisse erzielt werden.

In der Japanischen Patentanmeldung (Kokai 51 125 003) wird ein Verfahren zur Herstellung von Aldehyden durch Reaktion der entsprechenden Säure mit dem Produkt der Reaktion von Lithiumaluminiumhydrid mit einem sekundären Amin beschrieben. Die EP-A 0 087 298 beschreibt die Herstellung von Benzaldehyden ausgehend von Benzonitril durch Reaktion mit einem modifizierten Raney-Nickel.

Das Hinzufügen der Aluminiumhydridlösung zur Lösung der Verbindung der Formel (2) sollte vorzugsweise so langsam erfolgen, dass die Reaktionsmischung eine Temperatur von 50°C nicht übersteigt. Ein für die erfindungsgemässe Reduktion besonders geeigneter Temperaturbereich liegt zwischen 15 und 50°C. Auf Grund der hohen Hydrolyse- und Luftempfindlichkeit der Alkylaluminiumhydride wird die Reduktion unter Schutzgas, vorzugsweise Stickstoff, und in wasserfreiem, mit Schutzgas, insbesondere Stickstoff, gesättigten Lösungsmitteln durchgeführt.

Die so erhaltene Reaktionsmischung wird dann einer sauren Hydrolyse unterworfen, indem man sie langsam in eine wässrige Lösung einer anorganischen Säure überführt. Dabei sollte die Reaktionstemperatur in einem Bereich von 15 bis 50, vorzugsweise 30 bis 40°C gehalten werden. Als anorganische Säuren kommen beispielsweise Salzsäure, Schwefelsäure, Salpetersäure und Phosphorsäure in Betracht, wobei Salzsäure bevorzugt wird. Vorzugsweise verwendet man soviel Säure, dass nach der Hydrolyse der pH-Wert der Reaktionsmischung tief genug ist, um das Ausfallen von Aluminiumhydroxid zu verhindern.

Die Isolierung der Produkte der Verbindungen der Formel (1), kann dann nach an sich bekannten Verfahren geschehen, beispielsweise durch Abfiltrieren, nachdem gegebenenfalls das organische Lösungsmittel z.B. durch (Wasserdampf-)Destillation entfernt worden ist, Auskristallisieren oder Bilden von Additionsprodukten mit Natriumhydrogensulfit.

In einer bevorzugten Ausführungsform des erfindungsgemässen Verfahrens werden die Verbindungen der Formel (2), worin m und n 0 sind, in Toluol mit $(^iC_4H_9)_2AlH$ bei 15 bis 25°C umgesetzt, die erhaltene Reaktionsmischung anschliessend einer Hydrolyse in Gegenwart von Salzsäure bei einer Temperatur von 30 bis 40°C unterworfen und die Verbindungen der Formel (1) isoliert.

Vorzugsweise beträgt im erfindungsgemässen Verfahren das Molverhältnis von Verbindung der Formel (2) zu Aluminiumhydrid 1:1 bis 1:5. Je grösser der Ueberschuss an Aluminiumhydrid gewählt wird, desto tiefer sollte die Reaktionstemperatur bei der Reduktion gehalten werden, um Nebenreaktionen, insbesondere die Ueberreduktion der Cyanogruppen und die Reduktion der zwischenständigen Doppelbindung der Stilbenderivate, weitgehend zu vermeiden.

Besonders gute Ergebnisse werden mit dem erfindungsgemässen Verfahren erzielt, wenn das Molverhältnis zwischen 1:2 und 1:3 und die Reaktionstemperaturen bei der Reduktion im Bereich von 20 bis 25°C liegen.

Gegenstand der vorliegenden Erfindung sind des weiteren die aus der Reaktion erhältlichen neuen Zwischenprodukte der Formel

(3) 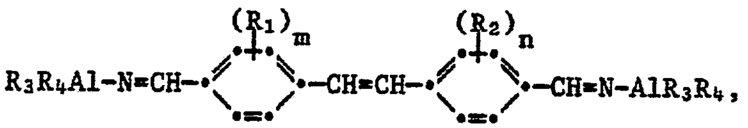

worin $R_1$, $R_2$, $R_3$, $R_4$, m und n die angegebenen Bedeutungen haben, welche, falls gewünscht, isoliert werden können und ein Verfahren zur Herstellung dieser Zwischenprodukte sowie die Verwendung dieser Zwischenprodukte und der Verbindungen der Formel (2) zur Herstellung von 4,4'-Stilbendialdehyden der Formel (1).

Die Herstellung der neuen Zwischenprodukte der Formel (3) erfolgt z.B. nach der oben beschriebenen Reduktion der Verbindungen der Formel (2) mit einem Aluminiumhydrid der Formel $R_3R_4AlH$ oder $M[R_3R_4AlH_2]$, worin $R_3$, $R_4$ und M die angegebenen Bedeutungen haben, in einem wasserfreien, inerten aprotischen organischen Lösungsmittel, wobei die Zwischenprodukte in praktisch reiner Form aus der Reaktionslösung gewonnen werden. Die Isolierung kann auf an sich bekannte Weise, wie z.B. Ausfällen, erfolgen.

Von diesen neuen Zwischenprodukten, die für die Herstellung von Verbindungen der Formel (1) verwendet werden können, kommt der Verbindung der Formel

(4) 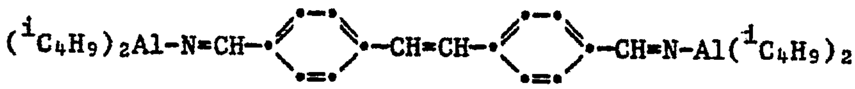

besondere Bedeutung zu. Vorzugsweise lässt sie sich herstellen, wenn man die Verbindung der Formel (2), worin m und n 0 sind, in Toluol bei 15 bis 25°C mit $(^iC_4H_9)_2AlH$ umsetzt.

Die Verbindungen der Formel (1) sind z.B. wertvolle Ausgangsverbindungen für die Herstellung von optischen Aufhellern.

Beispiel: In einem Sulfierkolben werden unter Stickstoffatmosphäre 230 g 4,4'-Stilbendinitril suspendiert in 1200 ml Toluol, das mit Stickstoff gesättigt worden ist, vorgelegt. Zu dieser Suspension werden 2008 ml einer 20%igen Lösung von Diisobutylaluminiumhydrid in Toluol innerhalb von 1,5 Stunden zugetropft, wobei sich eine klare, gelbliche, viskose Lösung bildet.

Man rührt noch 3,5 Stunden bei Raumtemperatur und tropft dann die Lösung innerhalb von 30 Minuten in eine Mischung von 1 l konzentrierter Salzsäure und 2 l Wasser. Die Temperatur des Reaktionsgemisches wird durch ein Eisbad zwischen 30 und 40°C gehalten.

Die so erhaltene gelbliche Suspension wird einer Wasserdampfdestillation unterworfen, um das Toluol zu entfernen. Der Rückstand im wässrigen Sumpf wird abfiltriert, mit Wasser neutral gewaschen und bei 80°C im Vakuumtrockenschrank getrocknet. Man erhält 226,2 g (95,7 %) Stilbendialdehyd vom Schmelzpunkt 168-170°C.

Gegebenenfalls kann das so erhaltene Produkt zur weiteren Reinigung im Toluol gelöst, ein- bis zweimal mit Bleicherde behandelt und durch Zugabe von Hexan ausgefällt werden. Man erhält dann 196,7 g (83,3 %) Stilbendialdehyd vom Schmelzpunkt 169-171°C.

Die vor der sauren Hydrolyse isolierbare Verbindung der Formel

(4) 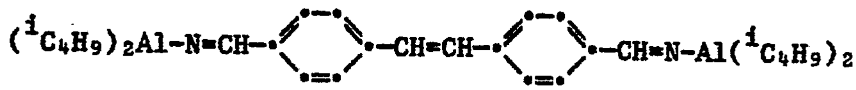

besitzt einen Schmelzpunkt von über 350°C. Die Struktur dieser Verbindungen wird durch das NMR-Spektrum bestätigt. Die Elementaranalyse ergab die folgenden Werte:

| | gefunden | berechnet |
|---|---|---|
| C | 70,5% | 74,7% |
| H | 9,0% | 9,4% |
| N | 5,1% | 5,4% |
| Al | 11,0% | 10,5% |

**Patentansprüche**

1. Verfahren zur Herstellung von Verbindungen der Formel

(1) 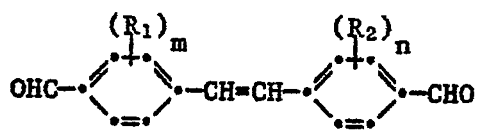 ,

worin $R_1$ und $R_2$ unabhängig voneinander Substituenten sind, die unter den unten definierten Reaktionsbedingungen nicht reduziert werden können, und m und n unabhängig voneinander 0,1 oder 2 sind, dadurch gekennzeichnet, dass man die Verbindungen der Formel

(2) 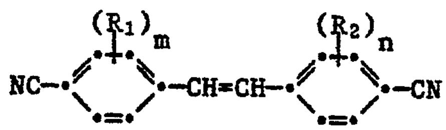 ,

worin $R_1$, $R_2$, m und n die angegebenen Bedeutungen haben, in einem wasserfreien, inerten, aprotischen, organischen Lösungsmittel mit einem Aluminiumhydrid umsetzt, die erhaltene Reaktionsmischung anschliessend einer sauren Hydrolyse unterwirft und die Verbindungen der Formel (1) isoliert.

2. Verfahren nach Anspruch 1, dadurch gekennzeichnet, dass als Lösungsmittel Kohlenwasserstoffe, Aether oder Gemische dieser Lösungsmittel verwendet werden.

3. Verfahren nach Anspruch 2, dadurch gekennzeichnet, dass als Lösungsmittel Alkane oder Cycloalkane mit je 5 bis 10 Kohlenstoffatome, Aromten, Dioxan, Tetrahydrofuran, Dibutyläther, Diäthyläther oder Gemische dieser Lösungsmittel verwendet werden.

4. Verfahren nach Anspruch 1, dadurch gekennzeichnet, dass als Aluminiumhydrid eine Verbindung der Formel $R_3R_4AlH$, $M[R_3R_4AlH_2]$ oder $MAlH_4$ verwendet wird, worin $R_3$ und $R_4$ unabhängig voneinander Alkyl mit je 1 bis 4 Kohlenstoffatomen sind, und M Lithium oder Natrium ist.

5. Verfahren nach Anspruch 4, dadurch gekennzeichnet, dass als Aluminiumhydrid $({}^{i}C_4H_9)_2AlH$, $Na[({}^{i}C_4H_9)_2AlH_2]$ oder $LiAlH_4$ verwendet wird.

6. Verfahren nach Anspruch 1, dadurch gekennzeichnet, dass die saure Hydrolyse in Gegenwart von Salzsäure, Schwefelsäure, Salpetersäure oder Phosphorsäure ausgeführt wird.

7. Verfahren nach Anspruch 1, dadurch gekennzeichnet, dass die Reduktion und die saure Hydrolyse bei einer Temperatur von 15 bis 50°C durchgeführt werden.

8. Verfahren nach den Ansprüchen 1 bis 7, dadurch gekennzeichnet, dass man die Verbindungen der Formel (2), worin m und n 0 sind, in Toluol bei 15 bis 25°C mit $({}^{i}C_4H_9)_2AlH$ umsetzt, die erhaltene Reaktionsmischung anschliessend einer Hydrolyse in Gegenwart von Salzsäure bei einer Temperatur von 30 bis 40°C unterwirft und die Verbindungen der Formel (1) isoliert.

9. Verfahren nach Anspruch 8, dadurch gekennzeichnet, dass das Molverhältnis von Verbindung der Formel (2) zu Aluminiumhydrid 1:1 bis 1:5 beträgt.

10. Zwischenprodukte der Formel

(3) 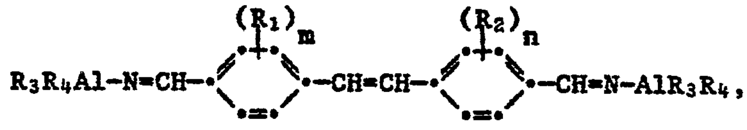

worin $R_1$, $R_2$, $R_3$, $R_4$ und m und n die in den Ansprüchen 1 und 4 angegebnen Bedeutungen haben.

11. Zwischenprodukt nach Anspruch 10 der Formel

(4) 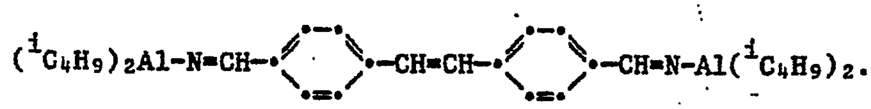

12. Verfahren zur Herstellung der Zwischenprodukte nach Anspruch 10, dadurch gekennzeichnet, dass man die Verbindungen der Formel (2), worin $R_1$, $R_2$, m und n die angegebenen Bedeutungen haben, in einem wasserfreien, inerten, aprotischen organischen Lösungsmittel mit einem Aluminiumhydrid der Formel $R_3R_4AlH$ oder $M[R_3R_4AlH_2]$, worin $R_3$, $R_4$ und M die in Anspruch 4 angegebenen Bedeutungen haben, umsetzt, und die Verbindungen der Formel (3) isoliert.

13. Verfahren nach Anspruch 12 zur Herstellung des Zwischenproduktes der Formel (4), dadurch gekennzeichnet, dass man die Verbindung der Formel (2), worin m und n 0 sind, in Toluol bei 15 bis 25 °C mit $(^iC_4H_9)_2AlH$ umsetzt und die Verbindung der Formel (4) isoliert.

14. Verfahren nach einem der Ansprüche 8 oder 12, dadurch gekennzeichnet, dass das Molverhältnis von Verbindung der Formel (2) zu Aluminiumhydrid zwischen 1:2 und 1:3 und die Reaktionstemperaturen bei der Reduktion im Bereich von 20 bis 25°C liegt.

15. Verwendung der Verbindungen der Formeln (2) und (3), worin $R_1$, $R_2$, $R_3$, $R_4$, m und n die in den Ansprüchen 1 und 4 angegebenen Bedeutungen haben, zur Herstellung der Verbindung der Formel (1).

## Claims

1. A process for the preparation of a compound of the formula

(1) 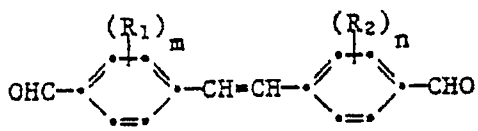

in which $R_1$ and $R_2$ are each independently of the other substituents that cannot be reduced under the reaction conditions defined below, and m and n are each independently of the other 0, 1 or 2, which process comprises reacting a compound of the formula

(2) 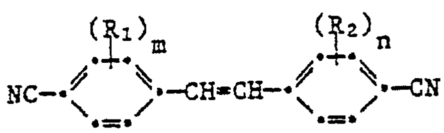

in which $R_1$, $R_2$, m and n have the given meanings, with an aluminium hydride, in an anhydrous, inert, aprotic organic solvent, thereafter subjecting the reaction mixture obtained to acid hydrolysis, and isolating the compound of the formula (1).

2. A process according to claim 1, wherein the solvent used is a hydrocarbon, an ether or a mixture of these solvents.

3. A process according to claim 2, wherein the solvent used is an alkane or cycloalkane, each of 5 to 10 carbon atoms, an aromatic compound, dioxane, tetrahydrofuran, dibutyl ether, diethyl ether, or a mixture of these solvents.

4. A process according to claim 1, wherein the aluminium hydride used is a compound of the formula $R_3R_4AlH$, $M[R_3R_4AlH_2]$ or $MAlH_4$, in which $R_3$ and $R_4$ are each independently of the other alkyl of 1 to 4 carbon atoms, and M is lithium or sodium.

5. A process according to claim 4, wherein the aluminium hydride is $(^iC_4H_9)_2AlH$, $Na[(^iC_4H_9)_2AlH_2]$ or $LiAlH_4$.

6. A process according to claim 1, wherein the acid hydrolysis is carried out in the presence of hydrochloric acid, sulfuric acid, nitric acid or phosphoric acid.

7. A process according to claim 1, wherein the reduction and the acid hydrolysis are carried out at a temperature from 15 to 50°C.

8. A process according to any one of claims 1 to 7, which comprises reacting the compound of formula (2), in which m and n are 0, with $(^iC_4H_9)_2AlH$ at 15 to 25°C in toluene, then subjecting the reaction mixture obtained to hydrolysis in the presence of hydrochloric acid at a temperature from 30 to 40°C, and isolating the compound of formula (1).

9. A process according to claim 8, wherein the molar ratio of the compound of formula (2) to aluminium hydride is 1:1 to 1:5.

10. An intermediate of formula

(3) 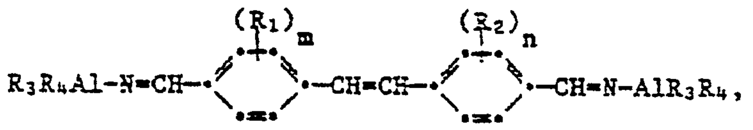

in which $R_1$, $R_2$, $R_3$, $R_4$, m and n are as defined in claims 1 and 4.

11. An intermediate according to claim 10 of formula

(4) 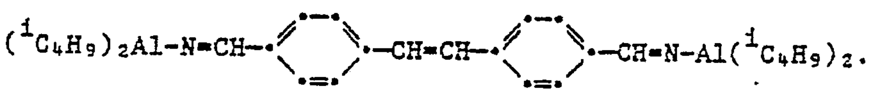

12. A process for the preparation of an intermediate according to claim 10, which comprises reacting the compound of formula (2), in which $R_1$, $R_2$, m and n have the given meanings, in an anhydrous, inert, aprotic organic solvent, with an aluminium hydride of formula $R_3R_4AlH$ or $M[R_3R_4AlH_2]$, in which $R_3$, $R_4$ and M are as defined in claim 4, and isolating the compound of formula (3).

13. A process according to claim 12 for the preparation of the intermediate of formula (4), which comprises reacting a compound of formula (2), in which m and n are 0, with $(^iC_4H_9)_2AlH$, in toluene and at temperature from 15 to 25°C, and isolating the compound of formula (4).

14. A process according to either claim 8 or claim 12, wherein the molar ratio of the compound of formula (2) to aluminium hydride is between 1:2 and 1:3 and the reaction temperatures during the reduction are in the range from 20 to 25°C.

15. Use of a compound of formula (2) or (3), in which $R_1$, $R_2$, $R_3$, $R_4$, m and n are as defined in claim 1 and 4, for the preparation of a compound of formula (1).

**Revendications**

1. Procédé pour la préparation de composés de formule

(1) 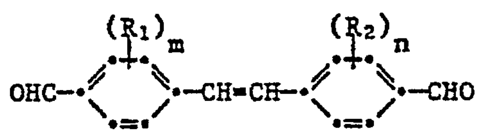 ,

dans laquelle $R_1$ et $R_2$ sont, indépendamment l'un de l'autre, des substituants qui ne peuvent pas être réduits dans les conditions réactionnelles définies ci-dessous, et m et n valent, indépendamment l'un de l'autre, 0, 1 ou 2, caractérisé en ce que l'on fait réagir avec un hydrure d'aluminium les composés de formule

(2) 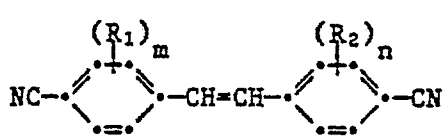 ,

dans laquelle $R_1$, $R_2$, m et n ont les significations données, dans un solvant organique aprotique inerte, anhydre, on soumet ensuite à une hydrolyse acide le mélange réactionnel obtenu et on isole les composés de formule (1).

2. Procédé selon la revendication 1, caractérisé en ce que l'on utilise, en tant que solvant, des hydrocarbures, des éthers ou des mélanges de ces solvants.

3. Procédé selon la revendication 2, caractérisé en ce que l'on utilise, en tant que solvant, des alcanes ou cycloalcanes ayant chacun de 5 à 10 atomes de carbone, des composés aromatiques, le dioxanne, le tétrahydrofuranne, l'éther dibutylique, l'éther diéthylique ou des mélanges de ces solvants.

4. Procédé selon la revendication 1, caractérisé en ce que l'on utilise, en tant qu'hydrure d'aluminium, un composé de formule $R_3R_4AlH$, $M[R_3R_4AlH_2]$ ou $MAlH_4$, dans lesquelles $R_3$ et $R_4$ sont, indépendamment l'un de l'autre, des groupes alkyle ayant chacun de 1 à 4 atomes de carbone, et M est le lithium ou le sodium.

5. Procédé selon la revendication 4, caractérisé en ce que l'on utilise, en tant qu'hydrure d'aluminium, $(i\text{-}C_4H_9)_2AlH$, $Na[i\text{-}C_4H_9)_2AlH_2]$ ou $LiAlH_4$.

6. Procédé selon la revendication 1, caractérisé en ce que l'on effectue l'hydrolyse acide en présence d'acide chlorhydrique, d'acide sulfurique, d'acide nitrique ou d'acide phosphorique.

7. Procédé selon la revendication 1, caractérisé en ce que l'on effectue la réduction et l'hydrolyse acide à une température de 15 à 50°C.

8. Procédé selon les revendications 1 à 7, caractérisé en ce que l'on fait réagir avec $(i\text{-}C_4H_9)_2AlH$ les composés de formule (2), dans lesquels m et n valent 0, dans du toluène, à 15–25°C, on soumet ensuite le mélange réactionnel à une hydrolyse en présence d'acide chlorhydrique, à une température de 30 à 40°C, et on isole les composés de formule (1).

9. Procédé selon la revendication 8, caractérisé en ce que la rapport molaire du composé de formule (2) à l'hydrure d'aluminium va de 1:1 à 1:5.

10. Produits intermédiaires de formule

(3) 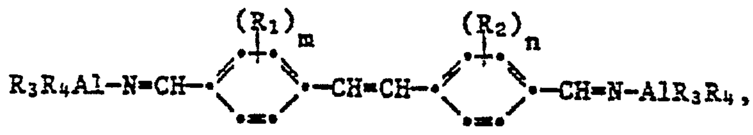

dans laquelle $R_1$, $R_2$, $R_3$, $R_4$ et m et n ont les significations données dans les revendications 1 et 4.

11. Produit intermédiaire selon la revendication 10, de formule

(4) 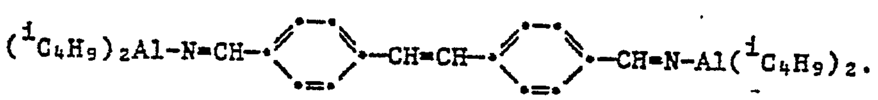

12. Procédé pour la préparation des produits intermédiaires selon la revendication 10, caractérisé en ce que l'on fait réagir les composés de formule (2), dans lesquels $R_1$, $R_2$, m et n ont les significations données, dans un solvant organique aprotique inerte, anhydre, avec un hydrure d'aluminium de formule $R_3R_4AlH$ ou $M[R_3R_4AlH_2]$, $R_3$, $R_4$ et M ayant les significations données dans la revendication 4, et on isole les composés de formule (3).

13. Procédé selon la revendication 12 pour la préparation du produit intermédiaire de formule (4), caractérisé en ce que l'on fait réagir avec $(i\text{-}C_4H_9)_2AlH$ le composé de formule (2), dans lequel m et n valent 0, dans du toluène, à 15–25°C, et on isole le composé de formule (4).

14. Procédé selon l'une des revendications 8 ou 12, caractérisé en ce que le rapport molaire du composé de formule (2) à l'hydrure d'aluminium est compris entre 1:2 et 1:3, et les températures de réaction lors de la réduction se situent dans la plage de 20 à 25°C.

15. Utilisation des composés de formules (2) et (3), dans lesquelles $R_1$, $R_2$, $R_3$, $R_4$, m et n ont les significations données dans les revendications 1 et 4, pour la préparation des composés de formule (1).